(19) Europäisches Patentamt / European Patent Office / Office européen des brevets

(11) **EP 4 548 846 A1**

(12) **EUROPEAN PATENT APPLICATION**

(43) Date of publication:
07.05.2025 Bulletin 2025/19

(21) Application number: 24209187.4

(22) Date of filing: 28.10.2024

(51) International Patent Classification (IPC):
*A61B 5/24* (2021.01)    *A61B 5/00* (2006.01)

(52) Cooperative Patent Classification (CPC):
**A61B 5/24; A61B 5/7203; A61B 5/7225; A61B 5/7235**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 30.10.2023 JP 2023185381

(71) Applicant: TDK Corporation
Tokyo 103-6128 (JP)

(72) Inventors:
• AKUSHICHI, Taiju
Chuo-ku, 103-6128 Tokyo (JP)
• TERAZONO, Yasushi
Chuo-ku, 103-6128 Tokyo (JP)
• SEKIHARA, Kensuke
Hachioji-shi, 190-0031 Tokyo (JP)

(74) Representative: Epping - Hermann - Fischer
Patentanwaltsgesellschaft mbH
Schloßschmidstraße 5
80639 München (DE)

(54) **MEASUREMENT APPARATUS**

(57) A measurement apparatus includes a signal sensor unit configured to measure a measurement signal in which a target signal and first noise are mixed and detect a first noise signal of which a level of a component of the target signal is lower than a predetermined value and which includes the first noise, and include one or more signal sensors; and a processing unit performing processing of reducing a component of the first noise included in measurement data by data partitioned factor analysis on the basis of the measurement data that is data of the measurement signal measured by the signal sensor unit and control data that is data of the first noise signal measured by the signal sensor unit.

FIG. 1

## Description

### CROSS-REFERENCE TO RELATED APPLICATION

Priority is claimed on Japanese Patent Application No. 2023-185381, filed on October 30, 2023, the content of which is incorporated herein by reference.

### BACKGROUND OF THE INVENTION

Field of the Invention

The present disclosure relates to a measurement apparatus.

Description of Related Art

In order to obtain favorable analytical results in various kinds of measurement, there is a need to eliminate various kinds of environmental noise which are superimposed on a desired signal.

For example, data partitioned factor analysis (PFA) has been made public as a technique of eliminating biologically originated environmental noise in magnetoencephalography (refer to Non-Patent Document 1 and Non-Patent Document 2, particularly p. 96-97).

In PFA, a signal measurement period is partitioned into two sections (temporal sections), such as a control section (for example, a so-called noise-only-section including no target signal) and a measurement section (a section including a target signal). Environmental noise components are estimated from the control section, and the environmental noise components are eliminated from the entire measurement data.

Regarding a specific example, in PFA, control data has been acquired by setting a section of a signal part in the middle (or in the vicinity of the middle) of a signal measurement period as a section of a target signal (measurement section) and a section on the past side sufficiently distant therefrom (for example, a section on the left side in the graph) or a section on the future side sufficiently distant therefrom (for example, a section on the right side in the graph) as a control section.

Patent Document 1 describes that noise is eliminated by active cancellation or signal processing is performed by signal space projection (SSP) using environmental noise data acquired separately from a measurement signal (refer to Patent Document 1).

[Patent Document]

[Patent Document 1] Published Japanese Translation No. 2008-538956 of the PCT International Publication

[Non-Patent Documents]

[Non-Patent Document 1] S. S. Nagarajan, H. T. Attias, K. E. Hild II and K. Sekihara, "A probabilistic algorithm for robust interference suppression in bioelectromagnetic sensor data", Stat. Med., vol. 26, no. 21, pp. 3886-3910, September 20, 2007.
[Non-Patent Document 2] K. Sekihara, and S. S. Nagarajan, "Electromagnetic Brain Imaging: A Bayesian Perspective", Springer, 2015/3/20, p. 75-82, p. 96-97
[Non-Patent Document 3] K. Sekihara, and S. S. Nagarajan, "Subspace-based interference removal methods for a multichannel biomagnetic sensor array", Journal of Neural Engineering 14(5) (2017) 051001
[Non-Patent Document 4] Kensuke Sekihara, "Bayesian Signal processing", Kyoritsu Shuppan, 2015/04/10, p. 65-72

### SUMMARY OF THE INVENTION

However, in such technologies in the related art described above, environmental noise components may remain without being completely eliminated when PFA is applied so that accuracy of reducing environmental noise components may become insufficient.

For example, in PFA, periodic waveform undulation may be present even after environmental noise components are eliminated using control data. Here, examples of periodic waveform undulation may include waveform undulation due to periodic noise derived from a natural frequency of a jig or the like of a sensor and a body motion (for example, heartbeats or the like) of a measurement subject, noise derived from a power source, magnetic noise from a surrounding motor, and the like.

In the technologies in the related art, since control data (control section) is selected by human visual inspection from a narrow region (signal measurement period), it may be unclear whether a target signal is completely included in the control section.

The present disclosure has been made in consideration of such circumstances, and an object thereof is to provide a measurement apparatus in which accuracy of reducing environmental noise components can be enhanced in PFA.

An aspect is a measurement apparatus including a signal sensor unit configured to measure a measurement signal in which a target signal and first noise are mixed and detect a first noise signal of which a level of a component of the target signal is lower than a predetermined value and which includes the first noise, and include one or more signal sensors; and a processing unit performing processing of reducing a component of the first noise included in measurement data by data partitioned factor analysis on the basis of the measurement data that is data of the measurement signal measured by the signal sensor unit and control data that is data of the first noise signal measured by the signal sensor unit.

According to the present disclosure, accuracy of reducing environmental noise components can be enhanced in PFA in the measurement apparatus.

BRIEF DESCRIPTION OF THE DRAWINGS

FIG. 1 is a view showing a schematic configuration of a measurement apparatus including an information processing device according to an embodiment.
FIG. 2 is a view showing a schematic configuration of the information processing device according to the embodiment.
FIG. 3 is a view schematically showing a flow of processing according to a first configuration example of the embodiment.
FIG. 4 is a view schematically showing a flow of processing according to a second configuration example of the embodiment.
FIG. 5 is a view schematically showing a flow of processing according to a third configuration example of the embodiment.
FIG. 6 is a view schematically showing a flow of processing according to a fourth configuration example of the embodiment.
FIG. 7 is a view showing an example of a waveform group of measurement data before noise elimination according to the embodiment.
FIG. 8 is a view showing an example of a waveform group of measurement data after noise elimination according to the embodiment.
FIG. 9 is a view showing an example of a waveform group of control data before noise elimination according to the embodiment.
FIG. 10 is a view showing an example of a waveform group of control data after noise elimination according to the embodiment.
FIG. 11 is a view showing an example of a waveform group of control data after preprocessing according to the embodiment.
FIG. 12 is a view showing an example of a waveform group in results of PFA processing according to the embodiment.
FIG. 13 is a view showing an example of a waveform of one channel before PFA processing according to the embodiment.
FIG. 14 is a view showing an example of a waveform of one channel in results of PFA processing according to the embodiment.
FIG. 15 is a view showing an example of a control section and a measurement section in PFA.
FIG. 16 is a view showing a schematic PFA algorithm.

DETAILED DESCRIPTION OF THE INVENTION

Hereinafter, an embodiment of the present disclosure will be described with reference to the drawings.

[Measurement apparatus]

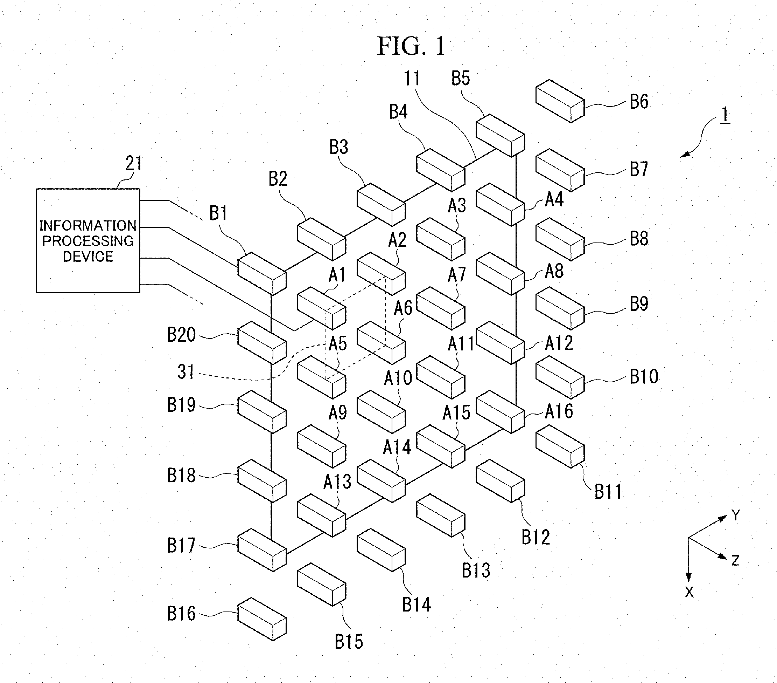

FIG. 1 is a view showing a schematic configuration of a measurement apparatus 1 including an information processing device 21 according to the embodiment.
FIG. 1 shows, for the sake of convenience, an XYZ orthogonal coordinate system that is a three-dimensional orthogonal coordinate system. The measurement apparatus 1 includes a plurality of (L) signal sensors A1 to AL, a plurality of (M) reference sensors B1 to BM, and the information processing device 21.
In the example of FIG. 1, a region in which the signal sensors A1 to AL are disposed is schematically indicated as a signal sensor disposition section 11.
For example, the measurement apparatus may also be referred to as a measurement system or the like.
Here, in the present embodiment, a case of L=16 and M=20 will be described as an example.
The number of signal sensors A1 to AL may be an arbitrary number equal to or larger than 2. In addition, the present embodiment describes a case in which there are a plurality of signal sensors A1 to AL, but the number of signal sensors may be one.
In addition, the number of reference sensors B 1 to BM may be an arbitrary number equal to or larger than 2. In addition, the present embodiment describes a case in which there are a plurality of reference sensors B 1 to BM, but the number of reference sensors may be one.
FIG. 1 also shows a measuring object 31. The measuring object may also be referred to as a measurement object or the like.
Regarding the measuring object 31, an arbitrary object may be used, or the entirety or a part of a living body may be used, for example. Regarding a specific example, a human heart, a human brain, or the like may be used the measuring object 31.
When the plurality of signal sensors A1 to AL are present, for example, a device integrally including the plurality of signal sensors A1 to AL (for example, a signal sensor unit) may be configured.
In addition, when the plurality of reference sensors B1 to BM are present, for example, a device integrally including the plurality of reference sensors B1 to BM (for example, a reference sensor unit) may be configured.
In addition, regarding another example, a device integrally including both the signal sensors A1 to AL and the reference sensors B1 to BM (for example, a sensor unit) may be configured.

<Disposition of signal sensors>

In the example of FIG. 1, the signal sensor disposition section 11 is a region having a square surface shape parallel to an XY plane.

In the example of FIG. 1, inside the signal sensor disposition section 11, sixteen signal sensors A1 to A16 are disposed at equal intervals in a direction parallel to one side of the square (a direction parallel to an X axis) and at equal intervals in a direction perpendicular to the direction (a direction parallel to a Y axis).

Specifically, four signal sensors A1 to A4 are disposed at equal intervals in a direction parallel to the Y axis, four signal sensors A5 to A8 are disposed at equal intervals in a direction parallel to the Y axis, four signal sensors A9 to A12 are disposed at equal intervals in a direction parallel to the Y axis, and four signal sensors A13 to A16 are disposed at equal intervals in a direction parallel to the Y axis.

In addition, the four signal sensors A1, A5, A9, and A13 are disposed at equal intervals in a direction parallel to the X axis, the four signal sensors A2, A6, A10, and A14 are disposed at equal intervals in a direction parallel to the X axis, the four signal sensors A3, A7, A11, and A15 are disposed at equal intervals in a direction parallel to the X axis, and the four signal sensors A4, A8, A12, and A16 are disposed at equal intervals in a direction parallel to the X axis.

In the example of FIG. 1, the equal intervals in a direction parallel to the X axis and the equal intervals in a direction parallel to the Y axis are the same intervals. In the example of FIG. 1, the plurality of signal sensors A1 to AL are disposed in an array shape.

For example, the plurality of signal sensors A1 to AL may also be referred to as a sensor array or the like.

Here, a form of disposing the signal sensors A1 to AL (entire disposition) is not particularly limited, and other forms may be used.

In addition, the disposition (for example, the position and the direction) of each of the signal sensors A1 to AL is not particularly limited, and diverse forms may be used. For example, in the example of FIG. 1, the direction of each of the signal sensors A1 to AL is shown to be the same, but the direction of each of the signal sensors A1 to AL may be arbitrary.

The signal sensor is a name for description and may also be referred to as other names such as a measurement sensor or (simply) a sensor, for example.

<Disposition of measuring object>

In the example of FIG. 1, when the signal sensor disposition section 11 is projected onto an XY plane while positional deviation parallel to a Z axis is disregarded, the measuring object 31 is disposed at the center (or in the vicinity thereof).

Here, a form of the disposition relationship between the measuring object 31 and the signal sensors A1 to AL is not particularly limited, and other forms may be used.

<Target signal>

Each of the signal sensors A1 to AL seeks for a desired signal caused by the measuring object 31 (for the sake of convenience, this will also be referred to as a target signal) as a measurement (detection) object. At this time, noise is superimposed on the target signal. For this reason, each of the signal sensors A1 to AL measures (detects) a signal in which the target signal and the noise are mixed (for the sake of convenience, this will also be referred to as a mixed signal). Further, each of the signal sensors A1 to AL obtains measurement results thereof (results of signal measurement).

In the present embodiment, for the sake of convenience, the mixed signal may be referred to as a measurement signal.

Regarding the target signal, a signal having an arbitrary physical quantity may be used. For example, a signal of magnetism, current, voltage, sound, light, or the like may be used.

In addition, regarding the target signal, for example, a signal caused by a living body (biological signal) may be used, or a signal caused by an object other than a living body may be used.

Regarding a specific example, when the measuring object 31 is a human heart, a magnetic signal (a signal of a magnetocardiogram) or an electrical signal (a signal of an electro-cardiogram) may be used as the target signal. Regarding another specific example, when the measuring object 31 is a human brain, a magnetic signal may be used as the target signal.

In addition, regarding another example, with sound as the target signal, it may be applied to a noise canceller of a speaker.

<Noise>

Here, for example, noise is caused by signals other than the target signal (interfering signal). For example, it may also be referred to as environmental noise or the like.

Regarding a specific example, the environmental noise may include noise of a natural frequency of a jig of a sensor array (for example, an array constituted of the plurality of signal sensors A1 to AL). For example, such noise may be emphasized by a human body motion (for example, heartbeats).

In addition, regarding a specific example, the environmental noise may include noise derived from a power source.

In addition, regarding a specific example, the environmental noise may include noise caused by rotation of a fan of an air conditioner (for example, operation of a motor) installed near the measurement apparatus 1.

In the present embodiment, in the information processing device 21, control data including such environmental noise is acquired, and components of the environmental noise from a measurement signal (measurement data) are reduced by PFA using the control data.

<Disposition of reference sensors>

In the example of FIG. 1, the reference sensors B1 to B20 are disposed in a manner of surrounding the signal sensor disposition section 11.

Specifically, on the negative side in a direction parallel to the X axis with respect to the signal sensor disposition section 11, six reference sensors B 1 to B6 are disposed at equal intervals in a direction parallel to the Y axis.

In addition, on the positive side in a direction parallel to the Y axis with respect to the signal sensor disposition section 11, six reference sensors B6 to B11 are disposed at equal intervals in a direction parallel to the X axis.

In addition, on the positive side in a direction parallel to the X axis with respect to the signal sensor disposition section 11, six reference sensors B11 to B 16 are disposed at equal intervals in a direction parallel to the Y axis.

In addition, on the negative side in a direction parallel to the Y axis with respect to the signal sensor disposition section 11, six reference sensors B16 to B20 and B 1 are disposed at equal intervals in a direction parallel to the X axis.

In the example of FIG. 1, the equal intervals in a direction parallel to the X axis and the equal intervals in a direction parallel to the Y axis are the same intervals. In the example of FIG. 1, the plurality of reference sensors B1 to BM are disposed around the signal sensors A1 to AL.

In the example of FIG. 1, each of the four reference sensors B2 to B5 parallel to the Y axis is disposed at the same position on the Y axis as each of the four signal sensors A1 to A4 parallel to the Y axis.

Similarly, each of the four reference sensors B12 to B 15 parallel to the Y axis is disposed at the same position on the Y axis as each of the four signal sensors A4, A3, A2, and A1 parallel to the Y axis.

In addition, in the example of FIG. 1, each of the four reference sensors B7 to B 10 parallel to the X axis is disposed at the same position on the X axis as each of the four signal sensors A1, A5, A9, and A13 parallel to the X axis.

Similarly, each of the four reference sensors B17 to B20 parallel to the X axis is disposed at the same position on the X axis as each of the four signal sensors A13, A9, A5, and A1 parallel to the X axis.

Here, a form of disposing the plurality of reference sensors B 1 to BM (entire disposition) is not particularly limited, and other forms may be used.

In addition, disposition (for example, the position, the direction) of each of the reference sensors B 1 to BM is not particularly limited, and diverse forms may be used. For example, in the example of FIG. 1, the direction of each of the reference sensors B 1 to BM is shown to be the same, but the direction of each of the reference sensors B 1 to BM may be arbitrary.

The reference sensor is a name for description and may also be referred to as other names such as a noise sensor or (simply) a sensor, for example.

<Reference noise>

Each of the reference sensors B 1 to BM measures (detects) noise superimposed on the target signal (noise signal). Further, each of the reference sensors B 1 to BM obtains measurement results thereof (results of noise measurement).

In the present embodiment, a case in which each of the reference sensors B 1 to BM measures noise without measuring the component of the target signal will be described. However, regarding another example, a configuration in which results of noise measurement by each of the reference sensors B 1 to BM include the component of the target signal to the extent that it does not cause practical problems may be used.

Here, in the present embodiment, for the sake of convenience, noise measured by the reference sensors B 1 to BM will also be referred to as reference noise, and a signal measured by the reference sensors B 1 to BM will also be referred to as a reference signal.

For example, the reference noise may have a noise component common to those of the environmental noise described above or may have a noise component different from those of the environmental noise described above.

Regarding a specific example, when the target signal is a magnetic signal, the reference noise may be noise caused by a geomagnetic signal. In addition, in this case, the reference noise may be noise caused by a magnetic signal caused by a person or other objects (for example, a train) other than a measurement object. In addition, regarding a specific example, when the target signal is an electrical signal, the reference noise may be electrical noise emitted from other circuits or the like in the vicinity of the measuring object 31.

In addition, for example, an electrical signal and a magnetic signal may be collectively treated as an electromagnetic signal (electromagnetic waves).

<Kind of signal sensor and kind of reference sensor>

When the plurality of signal sensors A1 to AL are used, for example, all the sensors may be the same kind (sensors measuring the same physical quantity), or different kinds of sensors (sensors measuring different physical quantities) may be included.

When the plurality of reference sensors B1 to BM are used, for example, all the sensors may be the same kind (sensors measuring the same physical quantity), or different kinds of sensors (sensors measuring different physical quantities) may be included.

In addition, regarding each of the reference sensors B1 to BM, for example, a sensor of the same kind as any of the signal sensors A1 to AL may be used, or a sensor of a kind different from that of the signal sensors A1 to AL may be used.

Regarding a specific example, when magnetic sensors are used as the signal sensors A1 to AL, magnetic sensors may be used as the reference sensors B1 to BM, or a combination of magnetic sensors and acceleration sensors may be used as the reference sensors B1 to BM.

<Connection between information processing device and each sensor>

In the example of FIG. 1, each of the signal sensors A1 to AL and the information processing device 21 are connected so as to be able to communicate with each other via wires or wirelessly. Further, the information processing device 21 can acquire measurement results (detection results) of each of the signal sensors A1 to AL.

In addition, each of the reference sensors B1 to BM and the information processing device 21 are connected so as to be able to communicate with each other via wires or wirelessly. Further, the information processing device 21 can acquire measurement results (detection results) of each of the reference sensors B1 to BM.

In the example of FIG. 1, regarding details of connection between the information processing device 21 and each of the sensors (the signal sensors A1 to AL and the reference sensors B1 to BM), illustration thereof is omitted.

Here, the present embodiment shows a case in which the information processing device 21 acquires measurement results of each of the sensors (the signal sensors A1 to AL and the reference sensors B1 to BM) by communicating with each of the sensors. However, regarding another example, a configuration in which measurement results of each of the sensors are stored once in a portable storage medium and then the measurement results are output to the information processing device 21 from the storage medium so that the information processing device 21 acquires the measurement results may be used.

<Information processing device>

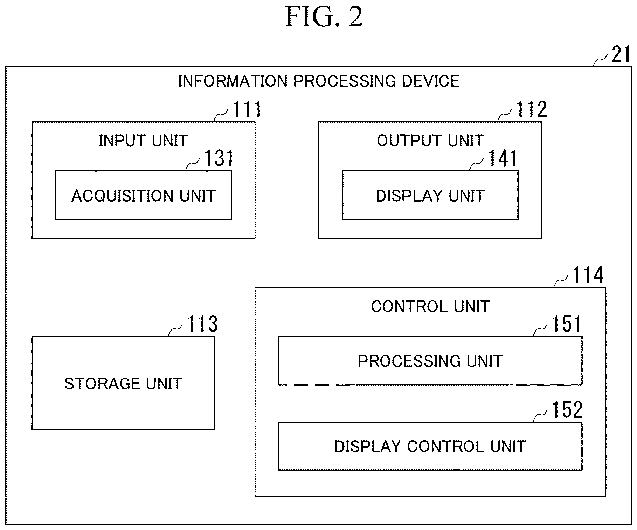

FIG. 2 is a view showing a schematic configuration of the information processing device 21 according to the embodiment.

The information processing device 21 includes an input unit 111, an output unit 112, a storage unit 113, and a control unit 114.

The input unit 111 includes an acquisition unit 131.

The output unit 112 includes a display unit 141.

The control unit 114 includes a processing unit 151 and a display control unit 152.

The input unit 111 performs inputting from the outside.

In the present embodiment, the input unit 111 inputs signals output from each of the sensors (the signal sensors A1 to AL and the reference sensors B1 to BM) (signals of measurement results). Regarding a specific example, the input unit 111 may input a signal transmitted from each of the sensors (the signal sensors A1 to AL and the reference sensors B1 to BM) upon reception of the signal or may input a signal stored in a portable storage device from the storage device.

In addition, for example, the input unit 111 may have an operation unit operated by a user and may be input information according to details of an operation performed by a user with respect to the operation unit.

The acquisition unit 131 acquires a signal input by the input unit 111.

The acquisition unit 131 may store an acquired signal in the storage unit 113.

Here, when a signal input by the input unit 111 is an analog signal, for example, the acquisition unit 131 may have an analog-to-digital (A/D) conversion function and convert the signal from an analog signal into a digital signal.

In addition, when the information processing device 21 is applied to real-time processing, the acquisition unit 131 acquires a signal in real time. Even when the information processing device 21 is not applied to real-time processing, the acquisition unit 131 may acquire a signal in real time.

Here, in the present embodiment, a case in which the acquisition unit 131 has a function of acquiring a signal measured by each of the signal sensors A1 to AL and a function of acquiring a signal measured by each of the reference sensors B1 to BM is described. However, regarding another example, these functions may be provided separately.

The output unit 112 performs outputting to the outside.

The display unit 141 performs display-outputting of information related to results of signal processing.

The display unit 141 has a screen such as a liquid crystal display (LCD) and performs display-outputting of information related to results of signal processing in the screen. Regarding another configuration example, the display unit 141 may perform print-outputting of information related to results of signal processing on paper.

The output unit 112 may have a function of performing outputting in other forms such as audio-outputting.

For example, the storage unit 113 has a storage device such as a memory and stores information.

For example, the storage unit 113 stores information such as input signals and processing results of the signals.

In addition, for example, the storage unit 113 stores information such as a control program.

The control unit 114 performs various kinds of processing and various kinds of control.

In the present embodiment, the control unit 114 has a processor such as a central processing unit (CPU) and performs various kinds of processing and various kinds of control when the processor executes the control program stored in the storage unit 113.

The processor includes a computation device performing various kinds of computation.

The processing unit 151 performs predetermined processing on the basis of signals of measurement results of the signal sensors A1 to AL. In the present embodiment, the predetermined processing is processing of reducing components of environmental noise included in a mixed signal (measurement signal) of a target signal and the environmental noise.

In addition, the processing unit 151 may perform predetermined processing on the basis of signals of measurement results of the signal sensors A1 to AL and signals of measurement results of the reference sensors B1 to BM.

In the present embodiment, reduction of a predetermined object component may also be referred to as elimination, for example. In this case, regarding the degree of reduction (elimination), for example, a form of completely eliminating the object component may be used, or a form of eliminating the object component to an extent that is not complete but practically effective may be used.

For example, regarding the degree of reducing components of environmental noise from a mixed signal (measurement signal), a practically effective degree and an arbitrary degree may be used, namely, a form of completely eliminating components of the environmental noise from the mixed signal may be used, or a form of eliminating components of the environmental noise from the mixed signal to a necessary extent may be used.

The display control unit 152 performs display-outputting of various kinds of information in the screen of the display unit 141.

In the present embodiment, the processing unit 151 eliminates components of environmental noise from a mixed signal (measurement signal) of a target signal and the environmental noise by performing processing of a PFA algorithm.

In the present embodiment, for the sake of convenience, data including the components of the environmental noise may be referred to as control data.

<Timing and period of control data>

In the present embodiment, respective timings (different timings) are used for a measurement timing of a measurement signal and a measurement timing of environmental noise (control data).

In the present embodiment, such respective timings mean that environmental noise (control data) is measured during a period when a measurement signal is not being measured. More specifically, a form of measuring environmental noise (control data) during a period when a target signal is not being measured (or during a period when the level of a target signal is lower than a predetermined value) is used. Regarding the predetermined value, a practically moderate value may be set.

Moreover, among the forms of such respective timings, an effect of reducing environmental noise components can be further enhanced when environmental noise which is the same as (or close to) the environmental noise included in a measurement signal can be measured as control data. For this reason, for example, a measurement timing of environmental noise (control data) may be immediately before or immediately after the measurement timing of a measurement signal.

Regarding a specific example, when a magnetic signal caused by the heart of a person (measurement subject) is measured, environmental noise (control data) may be measured at a timing in a state in which the heart of the person is away from a measurement position by a predetermined distance or longer (non-measurement state). At this timing, a target signal (in the present example, a magnetic signal caused by the heart) is not present, or the level of the target signal is lower than a predetermined value. For example, the target signal is smaller than the environmental noise.

In the present embodiment, a measurement subject represents a measurement object (person) and may also be referred to as a measuring subject or an examinee, for example.

In addition, the length of a measurement period of environmental noise (control data) is not particularly limited and may be a period having the same length or a period having a different length with respect to the length of a measurement period of a measurement signal, for example.

<Preprocessing>

The processing unit 151 may have a function of performing predetermined preprocessing with respect to control data.

Regarding the preprocessing, for example, processing of emphasizing components which become objects to be eliminated (components of environmental noise) from a measurement signal (measurement data) by PFA may be used. In a specific example, regarding the preprocessing, filtering processing of a band pass filter having a frequency band allowing the components to pass therethrough may be used, namely, filtering processing of extracting the components may be used.

<Noise elimination processing>

The processing unit 151 may have a function of performing predetermined noise elimination processing with respect to a measurement signal (measurement data) using measurement results of the reference sensors B1 to BM.

In addition, the processing unit 151 may have a function of performing predetermined noise elimination processing with respect to control data using measurement results of the reference sensors B1 to BM.

Here, regarding the noise elimination processing, for example, processing such as adaptive noise canceling (ANC) may be used (for example, refer to Non-Patent Document 3).

[Specific example of environmental noise elimination processing]

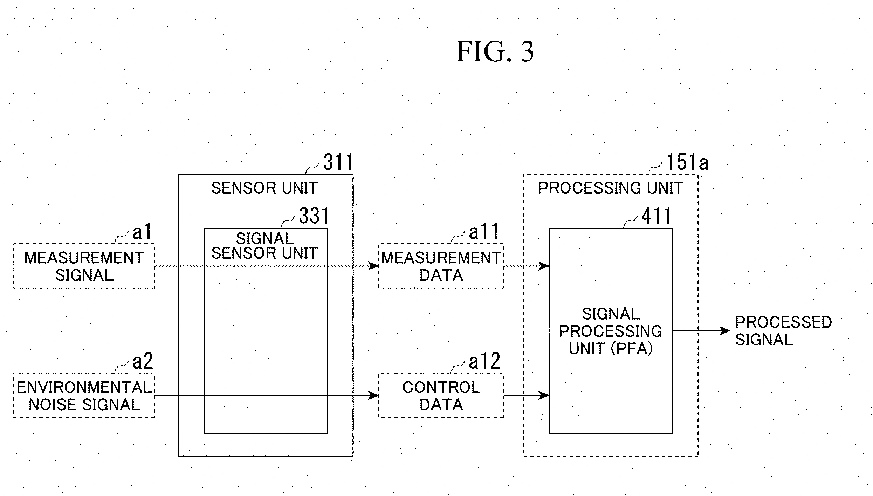

With reference to FIGS. 3 to 6, a specific example of environmental noise elimination processing performed by the information processing device 21 will be described.

In the present embodiment, for the sake of convenience, a part in which L signal sensors A1 to AL are combined will be referred to as a signal sensor unit 331 in the description, a part in which M reference sensors B1 to BM are combined will be referred to as a reference sensor unit 332 in the description, and a part in which the signal sensor unit 331 and the reference sensor unit 332 are combined will be referred to as a sensor unit 311 in the description.

In the configuration examples in which the reference sensors B1 to BM are not used, the reference sensor unit 332 (reference sensors B1 to BM) does not have to be provided.

Here, in the present example, the signal sensor unit 331 (signal sensors A1 to AL) is common to a measurement signal and an environmental noise signal. However, regarding another example, some or all thereof may differ between a measurement signal and an environmental noise signal.

In addition, in the present example, the reference sensor unit 332 (reference sensors B1 to BM) is common to a measurement signal and an environmental noise signal. However, regarding another example, some or all thereof may differ between a measurement signal and an environmental noise signal.

In addition, in the present example, the signal sensor unit 331 and the reference sensor unit 332 are separately provided.

<Specific example of processing according to first configuration example>

FIG. 3 is a view schematically showing a flow of processing according to a first configuration example of the embodiment.

FIG. 3 shows the sensor unit 311 including the signal sensor unit 331 and a processing unit 151a including a signal processing unit 411.

In the present example, the sensor unit 311 does not have to have the reference sensor unit 332 (reference sensors B1 to BM).

Here, the processing unit 151a is an example of the processing unit 151 shown in FIG. 2.

A specific example of processing in the present example will be described.

When a measurement signal a1 is measured by the signal sensor unit 331, measurement data a11 that is data of the measurement signal a1 is obtained.

In addition, when an environmental noise signal a2 is measured by the signal sensor unit 331 at a timing different from that of measuring the measurement signal a1, control data a12 that is data of the environmental noise signal a2 is obtained.

Here, in the present example, when the measurement signal a1 is measured by the signal sensor unit 331, a state in which the measuring object 31 is present at a predetermined measurement position is maintained.

Meanwhile, in the present example, when the environmental noise signal a2 is measured by the signal sensor unit 331, a state in which the measuring object 31 is not present at a predetermined measurement position is maintained.

Regarding a state in which the measuring object 31 is not present at a predetermined measurement position, for example, a state in which a signal component caused by the measuring object 31 (a component of a target signal) is not included in the environmental noise signal a2 (control data a12) or a state in which the level of the signal component is lower than a predetermined value even if the signal component is included in the environmental noise signal a2 (control data a12) is used.

The signal processing unit 411 reduces components of environmental noise included in the measurement data a11 by performing PFA processing using the measurement data a11 and the control data a12. Accordingly, a measurement signal (measurement data) in which components of environmental noise are reduced is obtained as a processed signal.

<Specific example of processing according to second configuration example>

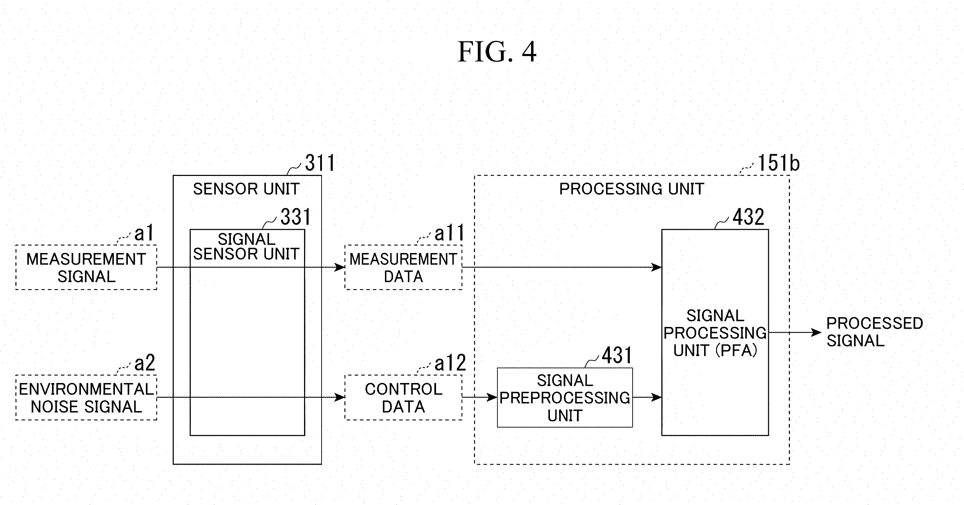

FIG. 4 is a view schematically showing a flow of processing according to a second configuration example of the embodiment.

FIG. 4 shows the sensor unit 311 including the signal sensor unit 331, and a processing unit 151b including a signal preprocessing unit 431 and a signal processing unit 432.

In the present example, the sensor unit 311 does not have to have the reference sensor unit 332 (reference sensors B1 to BM).

Here, the processing unit 151b is an example of the processing unit 151 shown in FIG. 2.

A specific example of processing in the present example will be described.

The present example is similar to the first configuration example except that the configuration of the processing unit 151b is different, and the same reference signs are applied to parts similar to those in the first configuration example.

In the present example, similar to the first configuration example, the measurement data a11 and the control data a12 are obtained.

The signal preprocessing unit 431 performs predetermined preprocessing with respect to the control data a12. The signal processing unit 432 reduces components of environmental noise included in the measurement data a11 by performing PFA processing using the measurement data a11 and the control data a12 after preprocessing. Accordingly, a measurement signal (measurement data) in which components of environmental noise are reduced is obtained as a processed signal.

In the second configuration example shown in FIG. 4, since components of environmental noise included in the control data a12 are emphasized when predetermined preprocessing is performed with respect to the control data a12 by the signal preprocessing unit 431, for example, compared to the first configuration example shown in FIG. 3, the accuracy of reducing environmental noise components by PFA can be enhanced.

<Specific example of processing according to third configuration example>

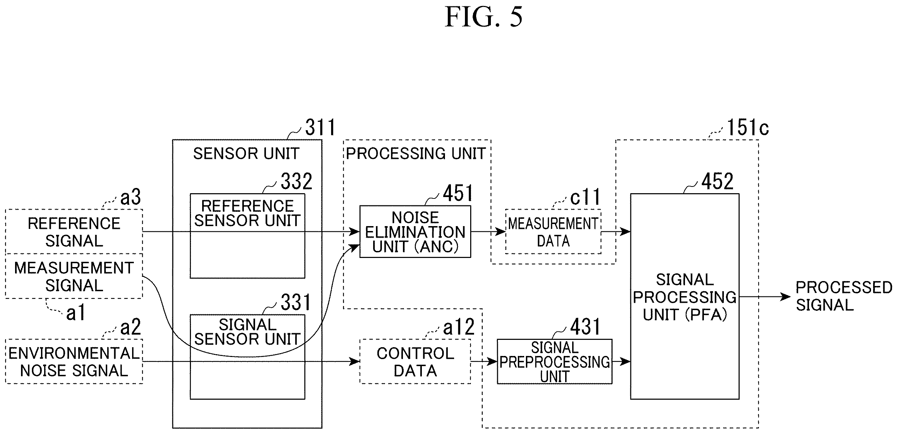

FIG. 5 is a view schematically showing a flow of processing according to a third configuration example of the embodiment.

FIG. 5 shows the sensor unit 311 including the signal sensor unit 331 and the reference sensor unit 332, and a processing unit 151c including the signal preprocessing unit 431, a noise elimination unit 451, and a signal processing unit 452.

Here, the processing unit 151c is an example of the processing unit 151 shown in FIG. 2.

A specific example of processing in the present example will be described.

The present example is similar to the second configuration example except that the reference sensor unit 332 is used and the configuration of the processing unit 151c is different (mainly, the noise elimination unit 451 is provided for the measurement signal a1), and the same reference signs are applied to parts similar to those in the second configuration example.

In the present example, similar to the second configuration example, preprocessing is performed for the control data a12 by the signal preprocessing unit 431.

The reference signal a3 related to the measurement signal a1 is measured by the reference sensor unit 332.

Here, in the present example, the reference signal a3 represents a signal measured by the reference sensor unit 332 when the measurement signal a1 is measured by the signal sensor unit 331.

The noise elimination unit 451 obtains measurement data c11 after noise elimination on the basis of measurement results of the measurement signal a1 by the signal sensor unit 331 (measurement data) and measurement results of the reference signal a3 by the reference sensor unit 332 (reference data) by performing processing of noise elimination with respect to the measurement data (in the present embodiment, elimination of components of reference noise).

Here, diverse techniques may be used as a noise elimination technique, and an ANC technique may be used, for example.

The signal processing unit 452 reduces components of environmental noise included in the measurement data c11 by performing PFA processing using the measurement data c11 after noise elimination and the control data a12 after preprocessing. Accordingly, a measurement signal (measurement data) in which components of environmental noise are reduced is obtained as a processed signal.

In the third configuration example shown in FIG. 5, since components of environmental noise included in the control data a12 become close to the environmental noise components included in the measurement data c11 when predetermined preprocessing is performed with respect to the control data a12 by the signal preprocessing unit 431, for example, compared to the first configuration example shown in FIG. 3, the accuracy of reducing environmental noise components by PFA can be enhanced.

In addition, in the third configuration example shown in FIG. 5, since noise elimination is performed with respect to measurement data by the noise elimination unit 451, for example, compared to the second configuration example shown in FIG. 4, the accuracy of reducing environmental noise components by PFA can be enhanced.

<Specific example of processing according to fourth configuration example>

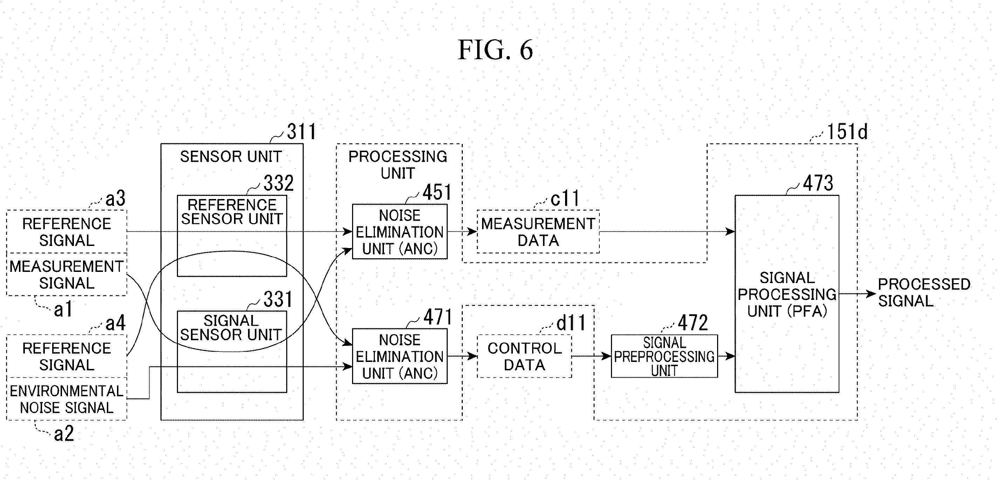

FIG. 6 is a view schematically showing a flow of processing according to a fourth configuration example of the embodiment.

FIG. 6 shows the sensor unit 311 including the signal sensor unit 331 and the reference sensor unit 332, and a processing unit 151d including the noise elimination unit 451, a noise elimination unit 471, a signal preprocessing unit 472, and a signal processing unit 473.

Here, the processing unit 151d is an example of the processing unit 151 shown in FIG. 2.

A specific example of processing in the present example will be described.

The present example is similar to the third configuration example except that the configuration of the processing unit 151d is different (mainly, the noise elimination unit 471 is provided for the environmental noise signal a2), and the same reference signs are applied to parts similar to those in the third configuration example.

In the present example, similar to the third configuration example, the measurement data c11 after noise elimination is obtained.

The reference signal a4 related to the environmental noise signal a2 is measured by the reference sensor unit 332. Here, in the present example, the reference signal a4 represents a signal measured by the reference sensor unit 332 when the environmental noise signal a2 is measured by the signal sensor unit 331.

The noise elimination unit 471 obtains control data d11 after noise elimination on the basis of measurement results of the environmental noise signal a2 by the signal sensor unit 331 (control data) and measurement results of the reference signal a4 by the reference sensor unit 332 (reference data) by performing processing of noise elimination with respect to the control data (in the present embodiment, elimination of components of reference noise).

Here, diverse techniques may be used as a noise elimination technique, and an ANC technique may be used, for example.

The signal preprocessing unit 472 performs predetermined preprocessing with respect to the control data d11 after noise elimination.

Here, regarding the preprocessing, for example, processing similar to the case of the second configuration example (and the third configuration example) may be performed or different processing may be performed.

The signal processing unit 473 reduces components of environmental noise included in the measurement data c11 by performing PFA processing using the measurement data c11 after noise elimination and the control data d11 after noise elimination and after preprocessing. Accordingly, a measurement signal (measurement data) in which components of environmental noise are reduced is obtained as a processed signal.

In the fourth configuration example shown in FIG. 6, since noise elimination is performed with respect to control data by the noise elimination unit 471, for example, compared to the third configuration example shown in FIG. 5, the accuracy of reducing environmental noise components by PFA can be enhanced.

[Example of waveform]

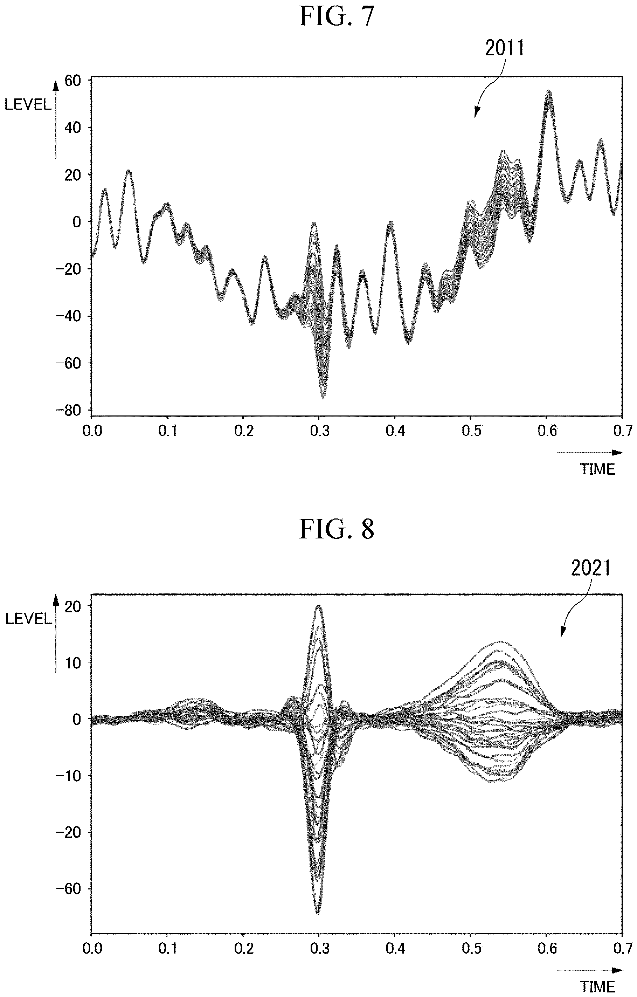

With reference to FIGS. 7 to 14, examples of waveforms of a signal processed by the information processing device 21 will be described. In the graph shown in each of FIGS. 7 to 14, the horizontal axis represents time, and the vertical axis represents level (for example, B[pT] that is the unit of magnetic flux density).

Each of the graphs shown in these diagrams shows the tendency of each waveform, which is not necessarily strict.

The present example shows an example of waveforms in the fourth configuration example shown in FIG. 6. The waveforms in the first to the third configuration examples shown in FIGS. 3 to 5 also have similar tendencies.

FIG. 7 is a view showing an example of a waveform group 2011 of measurement data before noise elimination according to the embodiment.

FIG. 8 is a view showing an example of a waveform group 2021 of measurement data after noise elimination according to the embodiment.

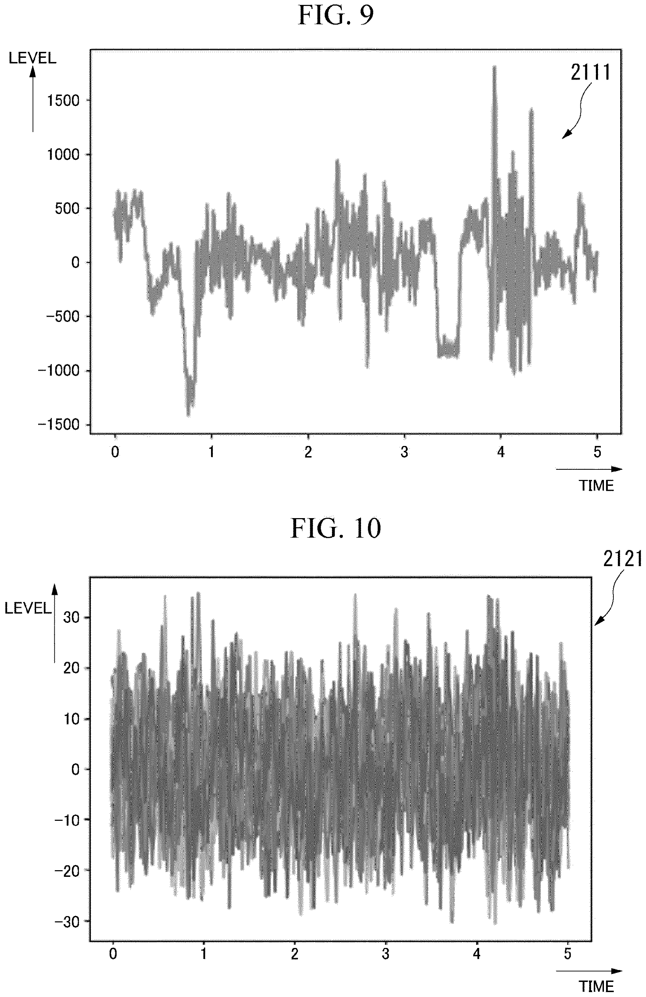

FIG. 9 is a view showing an example of a waveform group 2111 of control data before noise elimination according to the embodiment.

FIG. 10 is a view showing an example of a waveform group 2121 of control data after noise elimination according to the embodiment.

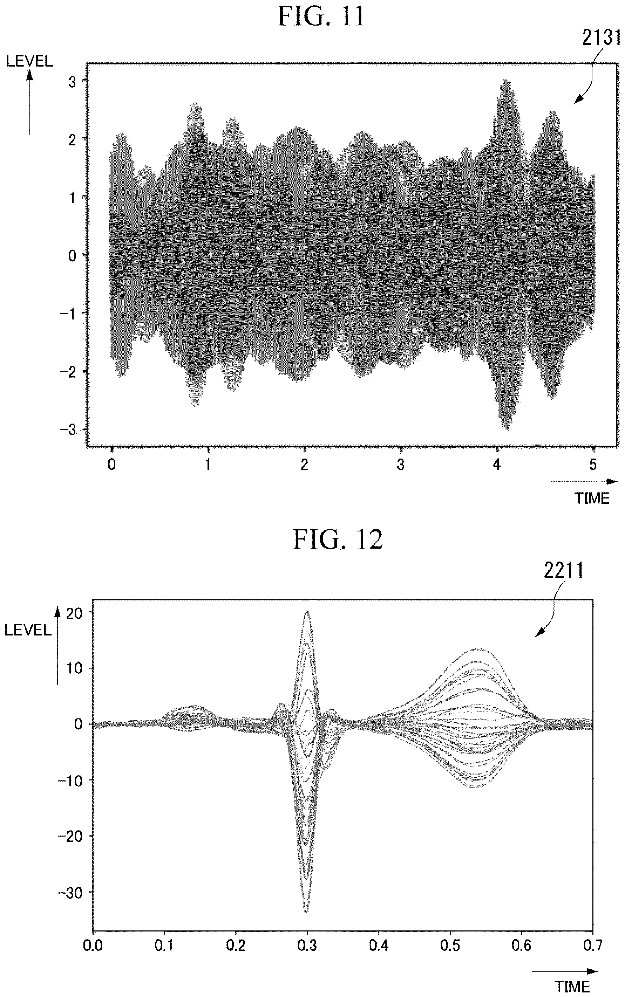

FIG. 11 is a view showing an example of a waveform group 2131 of control data after preprocessing according to the embodiment.

FIG. 12 is a view showing an example of a waveform group 2211 in results of PFA processing according to the embodiment.

FIG. 7 shows the waveform group 2011 in measurement results of the measurement signal a1 by the signal sensor unit 331 (measurement data). The waveform group 2011 is a waveform group before noise elimination is performed by the noise elimination unit 451.

The waveform group 2011 includes a plurality of (a plurality of channels of) waveforms measured by a plurality of signal sensors A1 to AL and average results of the plurality of waveforms.

FIG. 8 shows the waveform group 2021 in results of noise elimination performed by the noise elimination unit 451 (measurement data c11).

Here, in the present example, the waveforms of the measurement data before noise elimination include large noise (for example, noise due to a geomagnetic influence or the like), but components of such noise are reduced by noise elimination.

FIG. 9 shows the waveform group 2111 in measurement results of the environmental noise signal a2 by the signal sensor unit 331 (control data). The waveform group 2111 is a waveform group before noise elimination is performed by the noise elimination unit 471.

The waveform group 2111 includes a plurality of (a plurality of channels of) waveforms measured by a plurality of signal sensors A1 to AL and average results of the plurality of waveforms.

FIG. 10 shows the waveform group 2121 in results of noise elimination performed by the noise elimination unit 471 (control data d11). The waveform group 2121 is a waveform group before the preprocessing is performed by the signal preprocessing unit 472.

Here, in the present example, the waveforms of the control data before noise elimination include large noise due to a geomagnetic influence or the like, for example, but components of such noise are reduced by noise elimination.

FIG. 11 shows the waveform group 2131 in results of preprocessing performed by the signal preprocessing unit 472 (control data).

Here, in the present example, the waveforms of the control data before preprocessing also include noise components which are not objects to be reduced by PFA, for example, but such noise components are reduced by the preprocessing, namely, noise components which are objects to be reduced by PFA (environmental noise components) are emphasized.

FIG. 12 shows the waveform group 2211 in results of PFA performed by the signal processing unit 473 (processed signal).

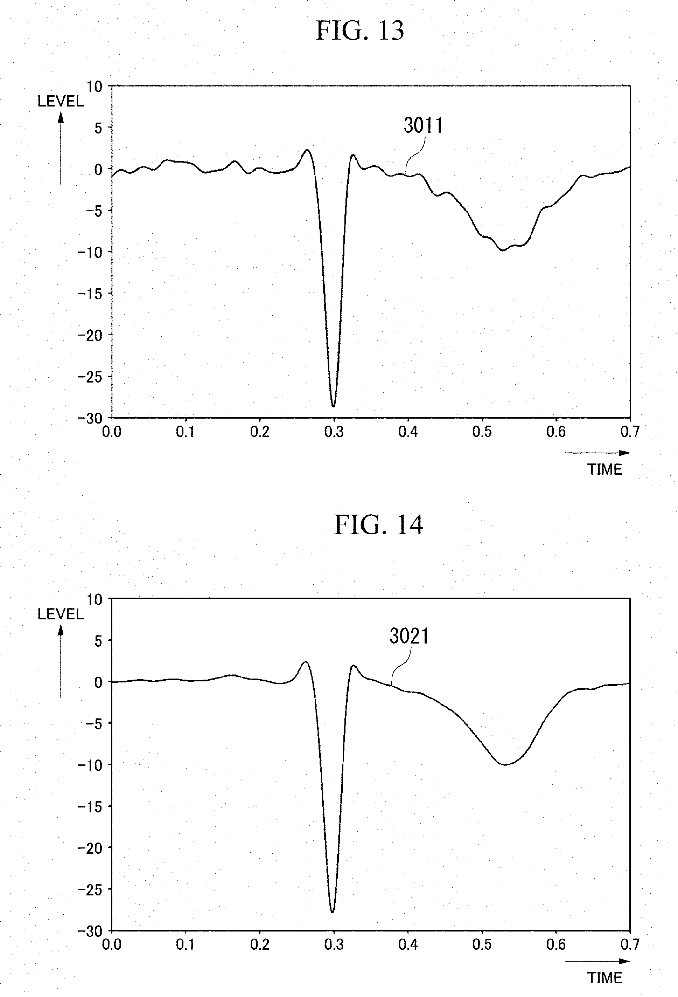

Here, FIGS. 13 and 14 each show an example of a waveform of one channel.

FIG. 13 is a view showing an example of a waveform 3011 of one channel before PFA processing according to the embodiment.

FIG. 14 is a view showing an example of a waveform 3021 of one channel in results of PFA processing according to the embodiment.

In the waveform 3021 in the results of PFA processing shown in FIG. 14, compared to the waveform 3011 before PFA processing shown in FIG. 13, components of environmental noise are reduced.

As shown in the examples of FIGS. 13 and 14, the waveform 3011 before PFA processing includes an undulation component, but such an undulation component is reduced in the waveform 3021 after PFA processing.

<Control section according to related art>

In the present embodiment, a control section and a measurement section are sections with different timings. However, regarding a comparative example, in the related art, PFA is performed by setting control sections on the left side and the right side of the waveform of a measurement signal (here, the waveform of a measurement signal before PFA) as shown in FIG. 12, namely, data of signals in a part of the section (ideally a section other than a target signal) of the measurement section is used as the control data. However, in such related art, there is a probability that measurement data may be included in the control data, and there are cases in which sufficient accuracy of PFA cannot be obtained.

In contrast, in the present embodiment, the accuracy of PFA processing can be enhanced.

[Overview of PFA]

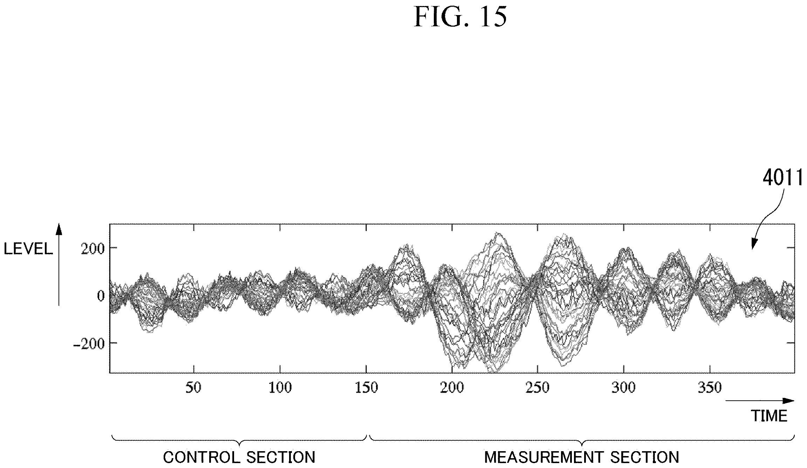

Here, with reference to FIGS. 15 to 16, overview of data partitioned factor analysis (PFA) will be described. For example, details of PFA are described in Non-Patent Document 1, and Non-Patent Document 2 particularly on p. 96-97.

PFA is an algorithm in which components of interfering signals (in the present embodiment, components of environmental noise) is eliminated from an object signal (in the present embodiment, a measurement signal) and a measurement object signal (in the present embodiment, a target signal) is estimated using the algorithm of Bayes factor analysis (BFA) twice. For example, details of Bayes factor analysis are described in Non-Patent Document 2 particularly on p. 75-82, and Non-Patent Document 4 particularly on p. 65-72.

FIG. 15 is a view showing an example of a control section and a measurement section in PFA.

In the graph shown in FIG. 15, the horizontal axis represents time, and the vertical axis represents level (for example, B[pT] that is the unit of magnetic flux density).

FIG. 15 shows a waveform group 4011 of signals (object signals) which become objects to be subjected to PFA processing.

The waveform group 4011 includes waveforms of a plurality of channels.

FIG. 15 shows an example of the control section used in PFA processing and an example of the measurement section. For example, the control section means a data section including only an interfering signal (environmental noise) and including no measurement object signal (target signal). However, regarding another example, the control section may include a measurement object signal (target signal) at a level larger than zero and smaller than a predetermined value.

An example of a mathematical expression representing a PFA data model will be described.

[Math. 1]

$$y_k = Au_k + Bv_k + \varepsilon : \text{measurement data}$$

$$y_k = Bv_k + \varepsilon : \text{control data}$$

Here, the factors are as follows.

$Au_k$: modeling of measurement object signal (vector)
$Bv_k$: modeling of interfering signal (vector)
$\varepsilon$: sensor noise (vector)
$y_k$: multi-channel sensor data (vector)
$k$: channel number of sensor (=1, 2, and so on)
A: mixing matrix
B: mixing matrix

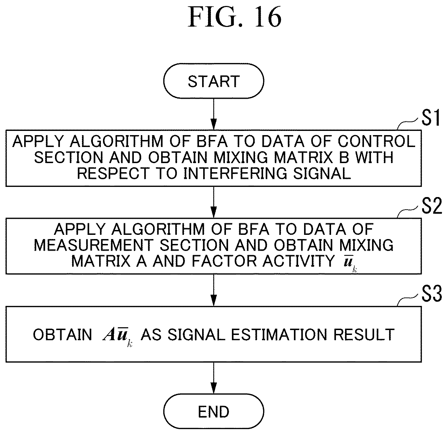

FIG. 16 is a view showing a schematic PFA algorithm.

In PFA, schematically, processing of (Step S1) to (Step S3) is performed in order.

In (Step S1), the algorithm of BFA is applied to data of the control section, and a mixing matrix B with respect to an interfering signal (in the present embodiment, environmental noise) is obtained.

In (Step S2), the algorithm of BFA is applied to data of the measurement section, and a mixing matrix A and a factor activity $u_k$ (here, the $u_k$ is a symbol with a bar thereabove) is obtained.

In (Step S3), $Au_k$ (here, the $u_k$ is a symbol with a bar thereabove) is obtained as a signal estimation result.

A comparison between PFA and SSP is performed. For example, details of SSP are described in Non-Patent Document 3.

In SSP, noise and a signal are separated using only spatial information. In addition, in SSP, the components (noise, signal) are separated as orthogonal components (orthogonal complement space).

Meanwhile, in PFA, noise and a signal are separated utilizing information of both the time and the space. For this reason, for example, PFA is also effective for signals of which, the position of a noise source and the position of a signal source are close to each other (signals in the same spatial area), such as noise caused by vibration of a jig.

In addition, in PFA, noise and a signal can be separated using an oblique basis. For this reason, in PFA, the components (noise, signal) can be separated without any excess or deficiency.

[Regarding foregoing embodiment]

As above, the measurement apparatus 1 according to the present embodiment includes the signal sensor unit 331 and the processing unit (in the example of FIG. 2, the processing unit 151).

The signal sensor unit 331 has one or more signal sensors (in the present embodiment, the signal sensors A1 to AL) acquiring signals and measures a signal (measurement signal) measured in a state in which a target signal and environmental noise are mixed.

The processing unit executes processing of eliminating environmental noise components by PFA using a measurement signal acquired by the signal sensor unit 331 as the measurement data and also using an environmental noise signal acquired by the signal sensor unit 331 separately from the measurement signal as the control data.

Regarding a configuration example, in the measurement apparatus 1 according to the present embodiment, the signal sensor unit 331 includes one or more signal sensors (in the present embodiment, the signal sensors A1 to AL). The signal sensor unit 331 has a function of measuring a measurement signal in which a target signal and first noise (in the present embodiment, environmental noise) are mixed and a function of detecting a first noise signal of which the level of a component of the target signal is lower than a predetermined value and which includes the first noise.

In addition, in the measurement apparatus 1 according to the present embodiment, the processing unit performs processing of reducing a component of the first noise included in measurement data by data partitioned factor analysis (PFA) on the basis of the measurement data that is data of the measurement signal measured by the signal sensor unit 331 and control data that is data of the first noise signal measured by the signal sensor unit 331.

Therefore, in the measurement apparatus 1 according to the present embodiment, accuracy of reducing environmental noise components (here, the component of the first noise) can be enhanced in PFA.

In the measurement apparatus 1 according to the present embodiment, the processing unit performs preprocessing of emphasizing environmental noise components desired to be eliminated with respect to the acquired control data.

Regarding a configuration example, in the measurement apparatus 1 according to the present embodiment, the processing unit performs preprocessing of emphasizing components which become objects to be reduced with respect to the control data before the data partitioned factor analysis (PFA).

Therefore, in the measurement apparatus 1 according to the present embodiment, accuracy of reducing environmental noise components can be further enhanced in PFA.

In addition to the signal sensor unit 331 having one or more signal sensors (in the present embodiment, the signal sensors A1 to AL) acquiring signals, the measurement apparatus 1 according to the present embodiment includes the reference sensor unit 332 having one or more reference sensors (in the present embodiment, the reference sensors B1 to BM) acquiring reference noise (predetermined noise).

The processing unit executes preliminary noise elimination processing with respect to the measurement signal (measurement data) acquired by the signal sensor unit 331 based on the data acquired by the reference sensor unit 332.

Regarding a configuration example, in the measurement apparatus 1 according to the present embodiment, the reference sensor unit 332 includes one or more reference sensors (in the present embodiment, the reference sensors B1 to BM). The reference sensor unit 332 has a function of measuring a second noise signal (in the present embodiment, a reference signal during measurement of a measurement signal) including second noise.

In addition, in the measurement apparatus 1 according to the present embodiment, the processing unit performs processing of reducing a component of the second noise included in the measurement data on the basis of the second noise signal measured by the reference sensor unit 332 before the data partitioned factor analysis (PFA).

Therefore, in the measurement apparatus 1 according to the present embodiment, accuracy of reducing environmental noise components can be further enhanced in PFA.

In the measurement apparatus 1 according to the present embodiment, the processing unit executes preliminary noise elimination processing with respect to the control data acquired by the signal sensor unit 331 based on the data acquired by the reference sensor unit 332.

Regarding a configuration example, in the measurement apparatus 1 according to the present embodiment, the reference sensor unit 332 has a function of measuring a third noise signal including third noise (in the present embodiment, a reference signal during measurement of an environmental noise signal).

In addition, in the measurement apparatus 1 according to the present embodiment, the processing unit performs processing of reducing a component of the third noise included in control data on the basis of the third noise signal measured by the reference sensor unit 332 before the data partitioned factor analysis (PFA).

Therefore, in the measurement apparatus 1 according to the present embodiment, accuracy of reducing environmental noise components can be further enhanced in PFA.

In the measurement apparatus 1 according to the present embodiment, the reference sensor unit 332 includes one or more sensors of the same kind as the signal sensor unit 331 or sensors of different kinds.

Regarding a configuration example, in the measurement apparatus 1 according to the present embodiment, at least one reference sensor included in the reference sensor unit 332 is a sensor of the same kind with respect to at least one signal sensor included in the signal sensor unit 331.

Therefore, in the measurement apparatus 1 according to the present embodiment, any appropriate kinds of sensors can be used as the signal sensor and the reference sensor.

In this manner, in the measurement apparatus 1 according to the present embodiment, components of various kinds of environmental noise (components of interfering signals other than a target signal) can be accurately eliminated in PFA.

In the measurement apparatus 1 according to the present embodiment, for example, with regard to noise which is difficult to be eliminated by PFA, components of environmental noise can be accurately eliminated from a measurement signal (highly accurate noise elimination) utilizing data which has been acquired separately from a measurement signal (for example, data including no target signal, such as empty-room noise data, or data including a target signal at a small level) as the control data.

Here, in the present embodiment, for example, the measurement data and the control data are separated more clearly than technologies in the related art. Accordingly, it is conceivable that the accuracy of eliminating environmental noise components by PFA is enhanced.

In addition, in the measurement apparatus 1 according to the present embodiment, for example, elimination of environmental noise components can be realized with higher accuracy by performing preprocessing of emphasizing environmental noise components desired to be eliminated with respect to the control data. Regarding a specific example, in the present embodiment, elimination of environmental noise components can be realized more efficiently, including periodic noise as well by performing PFA using results of predetermined preprocessing performed with respect to an environmental noise signal as the control data.

In addition, in the measurement apparatus 1 according to the present embodiment, for example, environmental noise can be efficiently eliminated and highly accurate measurement can be performed by providing a reference sensor for measuring the reference noise (predetermined noise) and combining a noise elimination technique such as ANC with PFA.

Here, in the present embodiment, for example, a measurement form in which a measurement subject is not covered with a magnetic shield may be used, or a measurement form in which a measurement subject is covered with a magnetic shield may be used. For example, in an environment of a significant external magnetic field, it may be effective to reduce the external magnetic field using a magnetic shield.

In the present embodiment, for example, PFA can also be applied to magnetic measurement in an environment outside a magnetic shield by installing the reference sensors B1 to BM and using noise elimination such as ANC in combination.

In addition, in the present embodiment, a case in which a measurement object is a human (measurement subject) has been described. However, regarding another example, the measurement object may be an object other than a human.

A program for realizing a function of an arbitrary configuration part in an arbitrary device described above may be recorded in a computer readable recording medium, and a computer system may read and execute the program. It is assumed that the said "computer system" includes an operating system or hardware such as peripherals. In addition, "the computer readable recording medium" indicates a portable medium such as a flexible disk, a magneto-optical disk, a ROM, or a compact disc (CD)-read only memory (ROM), and a storage device such as a hard disk built into the computer system. Moreover, it is assumed that "the computer readable recording medium" includes those retaining a program for a certain period of time, such as a volatile memory inside the computer system that serves as a server or a client when the program is transmitted through a network such as the Internet or a communication line such as a telephone line. For example, the volatile memory may be a random-access memory (RAM). For example, a recording medium may be a non-transitory recording medium.

In addition, the foregoing program may be transmitted from a computer system in which this program is stored in a storage device or the like to another computer system via a transmission medium or transmission waves in a transmission medium. Here, "the transmission medium" transmitting a program indicates a medium having a function of transmitting information, for example, a network such as the Internet or a communication line such as a telephone line.

In addition, the foregoing program may be a program for realizing a part of the functions described above. Moreover, the foregoing program may be a so-called differential file which can realize the functions described above in combination with a program which has already been recorded in the computer system. The differential file may also be referred to as a differential program.

In addition, a function of an arbitrary configuration part in an arbitrary device described above may be realized by a processor. For example, each step of the processing in the embodiment may be realized by a processor operating based on information such as a program, and a computer readable recording medium storing information such as a program. Here, for example, in the processor, the function of each part may be realized by separate hardware, or the function of each part may be realized by integrated hardware. For example, the processor may include hardware, and the hardware may include at least one of a circuit for processing digital signals and a circuit for processing analog signals. For example, the processor may be configured using one or both of one or a plurality of circuit devices mounted on a circuit board or one or a plurality of circuit elements. An integrated circuit (IC) or the like may be used as the circuit device, and a resistor, a capacitor, or the like may be used as the circuit element.

Here, for example, the processor may be a CPU. However, the processor is not limited to the CPU. For example, various kinds of processors such as a graphics processing unit (GPU) or a digital signal processor (DSP) may be used. In addition, for example, the processor may be a hardware circuit using an application specific integrated circuit (ASIC). In addition, for example, the processor may be constituted of a plurality of CPUs or may be constituted of a hardware circuit using a plurality of ASICs. In addition, for example, the processor may be constituted of a combination of a plurality of CPUs and a hardware circuit using a plurality of ASICs. In addition, for example, the processor may include one or more of an amplifier circuit, a filter circuit, and the like for processing analog signals.

Although embodiments of the present disclosure have been described in detail above with reference to the drawings, specific configurations are not limited to the embodiments and other designs and the like may also be included without departing from the scope of the present disclosure.

[Appendix]

(Configuration Example 1) to (Configuration Example 5) will be described.

(Configuration Example 1)

A measurement apparatus includes a signal sensor unit configured to measure a measurement signal in which a target signal and first noise are mixed and detect a first noise signal of which a level of a component of the target signal is lower than a predetermined value and which includes the first noise, and include one or more signal sensors; and a processing unit performing processing of reducing a component of the first noise included in measurement data by data partitioned factor analysis on the basis of the measurement data that is data of the measurement signal measured by the signal sensor unit and control data that is data of the first noise signal measured by the signal sensor unit.

(Configuration Example 2)

In the measurement apparatus according to (Configuration Example 1), the processing unit performs preprocessing of emphasizing a component to be reduced from the measurement signal with respect to the control data before the data partitioned factor analysis.

(Configuration Example 3)

The measurement apparatus according to (Configuration Example 1) or (Configuration Example 2) further includes a reference sensor unit configured to measure a second noise signal including second noise and including one or more reference sensors. The processing unit performs processing of reducing a component of the second noise included in the measurement data on the basis of the second noise signal measured by the reference sensor unit before the data partitioned factor analysis.

(Configuration Example 4)

In the measurement apparatus according to (Configuration Example 3), the reference sensor unit is configured to measure a third noise signal including third noise. The processing unit performs processing of reducing a component of the third noise included in the control data on the basis of the third noise signal measured by the reference sensor unit before the data partitioned factor analysis.

Here, the following configurations may be used.

(Configuration Example 4A)

The measurement apparatus according to (Configuration Example 1) or (Configuration Example 2) further includes a reference sensor unit configured to measure a third noise signal including third noise and include one or more reference sensors.

The processing unit performs processing of reducing a component of the third noise included in the control data on the basis of the third noise signal measured by the reference sensor unit before the data partitioned factor analysis.

(Configuration Example 5)

In the measurement apparatus according to (Configuration Example 3) or (Configuration Example 4), at least one of the reference sensors included in the reference sensor unit is the same kind of sensor with respect to at least one of the signal sensors included in the signal sensor unit.

The following configuration may be used.

(Configuration Example 5A)

In the measurement apparatus according to (Configuration Example 3) or (Configuration Example 4), at least one of the reference sensors included in the reference sensor unit is a different kind of sensor with respect to at least one of the signal sensors included in the signal sensor unit.

EXPLANATION OF REFERENCES

1 Measurement apparatus
11 Signal sensor disposition section
21 Information processing device
31 Measuring object
111 Input unit
112 Output unit
113 Storage unit
114 Control unit
131 Acquisition unit
141 Display unit
151, 151a to 151d Processing unit
152 Display control unit
311 Sensor unit
331 Signal sensor unit
332 Reference sensor unit
411, 432, 452, 473 Signal processing unit
431, 472 Signal preprocessing unit
451, 471 Noise elimination unit
2011, 2021, 2111, 2121, 2131, 2211, 4011 Waveform group
3011, 3021 Waveform
A1 to AL Signal sensor
B1 to BM Reference sensor
a1 Measurement signal
a2 Environmental noise signal
a3, a4 Reference signal
a11, c11 Measurement data
a12, d11 Control data

**Claims**

**1.** A measurement apparatus comprising:

a signal sensor unit configured to measure a measurement signal in which a target signal and first noise are mixed and detect a first noise signal of which a level of a component of the target signal is lower than a predetermined value and which includes the first noise, and include one or more signal sensors; and
a processing unit performing processing of reducing a component of the first noise included in measurement data by data partitioned factor analysis on the basis of the measurement data that is data of the measurement signal measured by the signal sensor unit and control data that is data of the first noise signal measured by the signal sensor unit.

**2.** The measurement apparatus according to claim 1, wherein the processing unit performs preprocessing of emphasizing a component to be reduced from the measurement signal with respect to the control data before the data partitioned factor analysis.

**3.** The measurement apparatus according to claim 1 or 2 further comprising:

a reference sensor unit configured to measure a second noise signal including second noise and including one or more reference sensors,
wherein the processing unit performs processing of reducing a component of the second noise included in the measurement data on the basis of the second noise signal measured by the reference sensor unit before the data partitioned factor analysis.

**4.** The measurement apparatus according to claim 3, wherein the reference sensor unit is configured to measure a third noise signal including third noise, and
the processing unit performs processing of reducing a component of the third noise included in the control data on the basis of the third noise signal measured by the reference sensor unit before the data partitioned factor analysis.

**5.** The measurement apparatus according to claim 3 or 4, wherein at least one of the reference sensors included in the reference sensor unit is the same kind of sensor with respect to at least one of the signal sensors included in the signal sensor unit.

FIG. 1

EP 4 548 846 A1

16

# FIG. 2

INFORMATION PROCESSING DEVICE ~21

INPUT UNIT ~111

ACQUISITION UNIT ~131

OUTPUT UNIT ~112

DISPLAY UNIT ~141

STORAGE UNIT ~113

CONTROL UNIT ~114

PROCESSING UNIT ~151

DISPLAY CONTROL UNIT ~152

# FIG. 3

```
                    311                              151a
      ┌─────────────────────────┐        ┌─────────────────────────┐
      │      SENSOR UNIT         │        │   PROCESSING UNIT       │
      │              331         │        │              411        │
  a1  │   ┌─────────────────┐    │   a11  │   ┌─────────────────┐   │
┌──────────┐│    SIGNAL     │  ┌──────────┐│                   │
│MEASUREMENT│─→│ SENSOR UNIT │─→│MEASUREMENT│──→│                │
│  SIGNAL  │  │             │  │  DATA    │  │     SIGNAL       │       PROCESSED
└──────────┘  │             │  └──────────┘  │   PROCESSING     │──→    SIGNAL
              │             │                │   UNIT (PFA)     │
  a2          │             │   a12          │                 │
┌──────────┐  │             │  ┌──────────┐  │                 │
│ENVIRONMENTAL│─→│           │─→│ CONTROL  │──→│                │
│NOISE SIGNAL│  │           │  │  DATA    │  │                 │
└──────────┘  └─────────────┘  └──────────┘  └─────────────────┘
```

FIG. 4

# FIG. 5

EP 4 548 846 A1

FIG. 6

EP 4 548 846 A1

# FIG. 7

# FIG. 8

# FIG. 9

# FIG. 10

## FIG. 11

2131

## FIG. 12

2211

## FIG. 13

## FIG. 14

# FIG. 15

EP 4 548 846 A1

# FIG. 16

START

S1

APPLY ALGORITHM OF BFA TO DATA OF CONTROL
SECTION AND OBTAIN MIXING MATRIX B WITH
RESPECT TO INTERFERING SIGNAL

S2

APPLY ALGORITHM OF BFA TO DATA OF
MEASUREMENT SECTION AND OBTAIN MIXING
MATRIX A AND FACTOR ACTIVITY $\bar{u}_k$

S3

OBTAIN $A\bar{u}_k$ AS SIGNAL ESTIMATION RESULT

END

Europäisches
Patentamt

European
Patent Office

Office européen
des brevets

# EUROPEAN SEARCH REPORT

**Application Number**

EP 24 20 9187

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2022 044224 A (TDK CORP) 17 March 2022 (2022-03-17) * paragraphs [0008], [0022] - [0024], [0062] - [0074]; figures 1-15 * | 1-5 | INV. A61B5/24 A61B5/00 |
| A | US 2020/368491 A1 (POLTORAK ALEXANDER [US]) 26 November 2020 (2020-11-26) * paragraph [0208] - paragraph [0222] * | 1-5 | |
| A | US 6 195 576 B1 (JOHN ERWIN ROY [US]) 27 February 2001 (2001-02-27) * column 7, line 1 - column 14, line 41 * | 1-5 | |

TECHNICAL FIELDS
SEARCHED (IPC)

A61B

The present search report has been drawn up for all claims

| Place of search | Date of completion of the search | Examiner |
|---|---|---|
| Munich | 7 January 2025 | Gentil, Tamara |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
 
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 20 9187

This annex lists the patent family members relating to the patent documents cited in the above-mentioned European search report.
The members are as contained in the European Patent Office EDP file on
The European Patent Office is in no way liable for these particulars which are merely given for the purpose of information.

07-01-2025

| Patent document cited in search report | Publication date | Patent family member(s) | Publication date |
|---|---|---|---|
| JP 2022044224 A | 17-03-2022 | JP 7577485 B2<br>JP 2022044224 A | 05-11-2024<br>17-03-2022 |
| US 2020368491 A1 | 26-11-2020 | US 2020368491 A1<br>US 2024066262 A1 | 26-11-2020<br>29-02-2024 |
| US 6195576 B1 | 27-02-2001 | NONE | |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023185381 A

- JP 2008538956 W

**Non-patent literature cited in the description**

- **S. S. NAGARAJAN** ; **H. T. ATTIAS** ; **K. E. HILD II** ; **K. SEKIHARA**. A probabilistic algorithm for robust interference suppression in bioelectromagnetic sensor data. *Stat. Med.*, 20 September 2007, vol. 26 (21), 3886-3910
- **K. SEKIHARA** ; **S. S. NAGARAJAN**. Electromagnetic Brain Imaging: A Bayesian Perspective. Springer, 20 March 2015, 75-82, 96-97

- **K. SEKIHARA** ; **S. S. NAGARAJAN**. Subspace-based interference removal methods for a multi-channel biomagnetic sensor array. *Journal of Neural Engineering*, 2017, vol. 14 (5), 051001
- **KENSUKE SEKIHARA**. Bayesian Signal processing. Kyoritsu Shuppan, 10 April 2015, 65-72